# EUROPÄISCHE PATENTSCHRIFT

(11) **EP 0 323 651 B1**
(45) Veröffentlichungstag und Bekanntmachung des Hinweises auf die Patenterteilung: **23.03.1994**
(21) Anmeldenummer: 88121929.9
(22) Anmeldetag: 31.12.1988
(51) Int. Cl.: C07D 307/60

(54) **Verfahren zur Gewinnung von farbstabilem Maleinsäureanhydrid**
Process for the recovery of colour-fast maleic anhydride
Procédé de récupération d'anhydride maléique ayant une couleur stabilisé

(30) Priorität: 08.01.1988 DE 3800308
(43) Veröffentlichungstag der Anmeldung: 12.07.1989
(73) Patentinhaber: BASF Aktiengesellschaft, 67063 Ludwigshafen (DE)
(72) Erfinder: Michl, Gerhard, D-6700 Ludwigshafen (DE); Seubert, Rolf, Dr., D-6710 Frankenthal (DE); Schmidt, Johannes E., Dr., D-6700 Ludwigshafen (DE)

(56) Entgegenhaltungen:
- EP-A- 0 168 807
- CH-A- 371 443
- DE-B- 676 330
- GB-A- 771 993
- US-A- 4 260 546

## Beschreibung

Diese Erfindung betrifft ein Verfahren zur Gewinnung von farbstabilem Maleinsäureanhydrid durch fraktionierte Destillation von rohem Maleinsäureanhydrid.

Maleinsäureanhydrid (MSA) wird bekanntlich durch katalytische Dampfphasenoxidation von Benzol, Naphthenen, Butenen und Butan hergestellt. Außerdem gewinnt man es bei der Herstellung von Phthalsäureanhydrid (PSA) durch Oxidation von o-Xylol und/oder Naphthalin als Nebenprodukt. Man isoliert es aus dem rohen MSA üblicherweise durch fraktionierte Destillation, bei der es nach Kristallisation als weißes, praktisch reines MSA gewonnen wird.

Es hat sich jedoch gezeigt, daß das so erhaltene weiße, kristalline MSA bei längerem Stehen zu Verfärbungen neigt. Die Verfärbung tritt noch schneller ein, wenn man das MSA in geschmolzenem Zustand hält. Da derartige Verfärbungen bei der weiteren Verwendung des MSA mitunter erheblich stören, vor allem beim Einsatz für die Herstellung von z.B. ungesättigten Polyesterharzen und Alkydharzen, wurde u.a. schon vorgeschlagen, nicht farbstabiles MSA durch eine chemische Behandlung in hochreines und farbstabiles MSA zu überführen. So gibt man nach den Angaben der GB-PS 1 204 846 z.B. Dibenzylsulfid zum destillierten MSA. Aus der DE-OS 20 08 619 ist bekannt, daß man farbbeständige Ware erhält, wenn man flüssiges MSA über bestimmte anorganische Salze, z.B. Bariumchlorid leitet.

US-A 4 260 546 beschreibt ein Verfahren zur Verbesserung der Farbe von geschmolzenem Maleinsäureanhydrid, worin das Maleinsäureanhydrid mit Wasserstoffperoxid und/oder Schwefelsäure bei Temperaturen von 60 bis 202°C behandelt wird. Die besten Ergebnisse werden durch die gemeinsame Anwendung von Wasserstoffperoxid und Schwefelsäure erzielt.

CH-A 371 443 betrifft ein Verfahren zur Reinigung von rohem Maleinsäureanhydrid von verfärbenden Bestandteilen, worin das rohe, gasförmige Maleinsäureanhydrid bei Temperaturen von 300 bis 450°C mit freien Sauerstoff enthaltenden Gasen in Gegenwart eines nichtpolaren Adsorptionsmittels einer oxidativen Behandlung unterzogen wird. In Abwesenheit des nichtpolaren Adsorptionsmittels oder bei Temperaturen unter 300°C werden die verfärbenden Bestandteile des Maleinsäureanhydrids nicht oxidiert.

Die Behandlung von MSA mit chemischen Zusätzen der genannten Art ist technisch aufwendig und hat außerdem den Nachteil, daß weitere Verunreinigungen in MSA eingebracht werden. Deshalb wurde nach einem Verfahren gesucht, das es gestattet, hochreines und farbstabiles MSA unter Vermeidung dieser Nachteile zu erhalten.

Es wurde nun gefunden, daß man bei der Herstellung von MSA durch fraktionierte Destillation von rohem MSA ein hochreines und farbstabiles MSA erhält, wenn man das rohe MSA vor oder während der Destillation mit Sauerstoff oder sauerstoffhaltigen Gasen behandelt.

Nach dem neuen Verfahren geht man von einem rohen MSA aus, das auf bekannte Weise, z.B. durch katalytische Oxidation von Benzol, Naphthenen, Phenol, Furan, Buten-1, Buten-2, Butan, o-Xylol oder Naphthalin erhalten wurde. Besonders vorteilhafte Ergebnisse werden beim Einsatz eines rohen MSA erzielt, das auf an sich bekannte Weise aus den Abwässern der Phthalsäureanhydrid-Produktion durch Luftoxidation von o-Xylol oder Naphthalin, wie sie z.B. in der DE-PS 2 356 049 beschrieben ist, erhalten wird.

Das rohe MSA, welches man bei einer der genannten Synthesen gewinnt, hat einen MSA-Gehalt von 80 bis 99 Gew.%, wobei der Rest aus herstellungsbedingten Nebenprodukten, wie PSA, Citraconsäureanhydrid, Benzoesäure, Chinonen u.a. besteht. Das rohe MSA wird einer an sich bekannten fraktionierten Destillation unterworfen, die sowohl kontinuierlich als auch diskontinuierlich sein kann. Bei dieser Reindestillation werden vom rohen MSA Leichtsieder und Hochsieder abgetrennt, während man das zu isolierende reine MSA als flüssige oder dampfförmige Fraktion zweckmäßigerweise dem Seitenabzug einer Destillationskolonne entnimmt. Bei der aus zwei Kolonnen bestehenden Destillation wird das reine MSA als Kopfprodukt der zweiten Kolonne abgezogen.

Der erfindungsgemäße Schritt besteht nun darin, daß man das rohe MSA vor oder während der Destillation mit Sauerstoff oder sauerstoffhaltigen Gasen behandelt. Anstelle von Sauerstoff kann man auch Sauerstoff enthaltende Gase verwenden. Die Verwendung von Luft ist bevorzugt. Pro Mol Roh-MSA wendet man z.B. 0,15 bis 30, vorzugsweise 1 bis 15 Millimol Sauerstoff an. Die Sauerstoffbehandlung wird bei 40 bis 220°C, vorzugsweise bei 60 bis 180°C vorgenommen.

Die Behandlung des rohen MSA mit Sauerstoff geschieht zweckmäßigerweise durch Einleiten von Luft in das geschmolzene Roh-MSA. Zu diesem Zweck empfiehlt sich die Installation eines an sich üblichen Begasungsbehälters bzw. eines für die Durchmischung von Gas und Flüssigkeit geeigneten Mischgerätes zwischen dem Tank für das Roh-MSA und der Kolonne für die Reindestillation. Bei dieser Behandlung mit Sauerstoff wählt man im allgemeinen Temperaturen von 60 bis 180°C. Diese Sauerstoffbehandlung kann bei Atmosphärendruck sowie auch unter leichtem Unter- oder überdruck durchgeführt werden. Man kann die Luft aber auch zusammen mit dem Roh-MSA in die Destillationskolonne einleiten. Die Fraktionierung zur Abtrennung der Leicht- und Hochsieder und die erfindungsgemäße Reinigung des MSA finden dabei gleichzeitig statt. Die Temperaturen in der Destillationskolonne liegen etwa bei 100 bis 180°C. In der Destillationskolonne wird die Sauerstoffbehandlung üblicherweise unter Vakuum durchgeführt.

Nach dem Verfahren der Erfindung erhält man überraschenderweise ein MSA von hervorragender Qualität, das sich besonders durch seine hohe Farbstabilität auszeichnet.

### Beispiel 1 (Vergleich)

2,2 kg/h rohes MSA, das auf an sich bekannte Weise aus den Abwässern einer Phthalsäureanhydrid-Produktion durch Luftoxidation von o-Xylol erhalten wurde, mit der Zusammensetzung 87,5 Gew.% MSA, 3,0 Gew.% Citraconsäureanhydrid, 2,5 Gew.% Phthalsäureanhydrid, 3,5 Gew.% Benzoesäure und 3,5 Gew.% sonstige Verunreinigungen wurden mit einer Zulauftemperatur von 90°C kontinuierlich auf den 10. Boden einer Kolonne gegeben. Die Kolonne hatte einen Durchmesser von 80 mm und enthielt 50 Böden. Sie wurde bei einem Druck von 100 mbar am Kopf betrieben. Am Kolonnenkopf wurde eine Gesamtdestillatmenge von 7,5 kg/h (Kondensationstemperatur 70°C) gemessen, wobei 0,07 kg/h als Kopfabzug entnommen und 7,4 kg/h als Rücklauf auf den Kolonnenkopf gegeben wurden. Der Kopfabzug enthielt 99,98 Gew.% MSA. Am Kolonnensumpf wurden 0,30 kg/h (Temperatur 170°C) abgezogen. Der Sumpfaustrag enthielt 27,0 Gew.% MSA; 18,0 % Citraconsäureanhydrid; 27,5 Gew.% Benzoesäure; 23,0 Gew.% Phthalsäureanhydrid und sonstige Verunreinigungen, wie hochsiedende Kohlenwasserstoffe, hochmolekulare organische Säuren und Zersetzungsprodukte. Aus einem am 40. Boden angebrachten Seitenabzug wurden 1,8 kg/h flüssiges MSA bei einer Temperatur von 130°C abgezogen. Es hatte eine Schmelzfarbzahl von 5 bis 10 Hazen. Im Lagertank wurde nach ca. 48 Stunden eine Schmelzfarbenzahl von 40 bis 60 Hazen festgestellt.

### Beispiel 2

Die Destillation des Roh-MSA wurde wie in Beispiel 1 beschrieben durchgeführt, wobei jedoch gleichzeitig mit dem Roh-MSA 7 l/h Luft in die Destillationskolonne eingeleitet wurden. Das aus dem Seitenabzug der Kolonne gewonnene MSA hatte eine Schmelzfarbzahl von 5 bis 10 Hazen. Auch nach einer Lagerzeit von mehreren Tagen war keine Verschlechtung der Schmelzfarbzahl festzustellen.

## Patentansprüche

1. Verfahren zur Gewinnung von farbstabilem Maleinsäureanhydrid durch fraktionierte Destillation von rohem Maleinsäureanhydrid, dadurch gekennzeichnet, daß man das rohe Maleinsäureanhydrid vor oder während der Destillation mit Sauerstoff oder sauerstoffhaltigen Gasen bei 40 bis 220°C behandelt.

2. Verfahren nach Anspruch 1, dadurch gekennzeichnet, daß man auf 1 Mol rohes Maleinsäureanhydrid 0,15 bis 30 Millimol Sauerstoff anwendet.

3. Verfahren nach Anspruch 1, dadurch gekennzeichnet, daß man als sauerstoffhaltiges Gas Luft verwendet.

4. Verfahren nach Anspruch 1, dadurch gekennzeichnet, daß das rohe Maleinsäureanhydrid aus dem Abwasser der Abgaswäsche einer Phthalsäureanhydrid-Produktion durch Luftoxidation von o-Xylol oder Naphthalin erhalten wurde.

## Claims

1. A process for isolating colour-stable maleic anhydride by fractional distillation of crude maleic anhydride, which comprises treating the crude maleic anhydride with oxygen or oxygen-containing gases at from 40 to 220°C before or during the distillation.

2. A process as claimed in claim 1, wherein from 0.15 to 30 millimol of oxygen are used per mole of crude maleic anhydride.

3. A process as claimed in claim 1, wherein air is used as oxygen-containing gas.

4. A process as claimed in claim 1, wherein the crude maleic anhydride was obtained from the exhaust gas scrubber effluent from phthalic anhydride production by air oxidation of o-xylene or naphthalene.

## Revendications

1. Procédé de récupération d'un anhydride maléique de couleur stable par distillation fractionnée d'anhydride maléique brut, caractérisé en ce que l'on traite l'anhydride maléique brut, avant ou pendant la distillation, avec de l'oxygène ou des gaz contenant de l'oxygène à 40-220°C.

2. Procédé selon la revendication 1, caractérisé en ce qu'on utilise 0,15 à 30 millimoles d'oxygène pour 1 mole d'anhydride maléique brut.

3. Procédé selon la revendication 1, caractérisé en ce qu'on utilise comme gaz contenant de l'oxygène de l'air.

4. Procédé selon la revendication 1, caractérisé en ce que l'anhydride maléique brut a été obtenu à partir des eaux résiduaires du lavage des gaz résiduaires de la production d'anhydride phtalique par oxydation a l'air du o-xylène ou du naphtalène.
